# EUROPEAN PATENT SPECIFICATION

(11) **EP 1 831 167 B1**
(45) Date of publication and mention of the grant of the patent: **13.07.2011**
(21) Application number: 04816687.0
(22) Date of filing: 31.12.2004
(51) Int. Cl.: C07D 213/09

(54) **LIQUID PHASE PROCESS FOR THE SYNTHESIS OF ANNULATED PYRIDINES OVER ZEOLITE TYPE MOLECULAR SIEVE CATALYSTS**
FLÜSSIGPHASENVERFAHREN ZUR SYNTHESE ANELLIERTER PYRIDINE ÜBER MOLSIEBKATALYSATOREN VOM ZEOLITHTYP
PROCÉDÉ EN PHASE LIQUIDE POUR LA SYNTHÈSE DE PYRIDINES ANNELÉES SUR DES CATALYSEURS SOUS FORME DE TAMIS MOLÉCULAIRES DE TYPE ZÉOLITE

(43) Date of publication of application: 12.09.2007
(73) Proprietor: Council of Scientific and Industrial Research, New Delhi 110 001 (IN)
(72) Inventor: KULKARNI, Shivanand, Janardan, Hyderabad 500 007,Andhra Pradesh (IN); KANDEPI, Veera, Venkata, Krishna, Mohan, Hyderabad 500 007, Andhra Pradesh (IN); NAMA, Narender, Hyderabad 500 007, Andhra Pradesh (IN); RAGHAVAN, Kondapuram, Vijaya, Hyderabad 500 007, Andhra Pradesh (IN)
(74) Representative: Maiwald, Walter
(86) International application number: PCT/IN2004/000442
(87) International publication number: WO 2006/070403

(56) References cited:
- WO-A-02/079166
- US-A- 4 220 783
- THUMMEL R P ET AL: "PREPARATION AND PROPERTIES OF ANNELATED PYRIDINES" JOURNAL OF ORGANIC CHEMISTRY, AMERICAN CHEMICAL SOCIETY. EASTON, US, vol. 42, no. 16, 1977, pages 2742-2747, XP002094916 ISSN: 0022-3263 cited in the application
- TCHITCHIBABIN: "Sur les réactions de condensation des aldéhydes et cétones avec l'ammoniaque en bases pyridiques. Condensations avec les cétones cycliques" BULL. SOC. CHIM. FR., vol. 6, 1939, pages 522-533, XP009052662

## Description

### Field of the invention

The present invention relates to an improved liquid phase process for the synthesis of annulated pyridines (fused pyridines) over molecular sieve catalysts. In particular, the present invention provides a process for producing 1,2,3,4,5,6,7,8-octahydroacridine and octahydrophenanthridine by reacting cyclohexanone and formaldehyde with ammonia in liquid phase over molecular sieve catalysts with high yields and selectivity. The present invention provides a non-corrosive, eco-friendly process, where the life time of the catalyst is longer, it can be recycled and reused for many times, less wastage of compounds (e.g. high atom selectivity) and high selectivity of the products.

### Background of the invention

Annulated pyridines like 9-amino-5,6,7,8-tetrahydroacridine (tacrine) are drug or drug intermediates for various diseases like Alzheimer's disease, which is the most common cause of dementia in old people. Many methods of producing pyridine bases are known, for example reacting an aliphatic aldehyde and/or ketone with ammonia in gaseous phase using a solid acid catalyst such as amorphous aluminosilicate and the like (Japanese Patent Application Kokai (Laid-open) No.63176/76, Japanese Patent Publication No.3 41546/71 and 32790/69). Crystalline aluminosilicate, called zeolite is used as the catalyst for producing pyridine bases from an aliphatic aldehyde and ketone (or formaldehyde) and ammonia (US Patent 4220783, Japanese Patent Application Kokai (Laid-open) No.38362/85, Indian Patent Nos. IN-185390, IN-185654). According to these processes one ring pyridine compounds are produced but fused ring heterocyclics have not yet been reported over zeolite molecular sieve catalysts. Increasing applications of these annelated pyridines demand an eco-friendly, economical and free handling process. The present invention provides an eco-friendly and economical process for synthesis of a variety of these compounds.

The synthesis of octahydroacridine and octahydrophenanthridine was carried out using homogenous catalyst like ammonium acetate and ammonium hydroxide but the yield were lower than 54% and usual disadvantages of homogenous catalysis were observed (J.Org.Chem.42 (No. 16), p2742 (1977). The synthesis of octahydrophenanthridine was also carried out using p-tolune sulfonic acid and POCl₃ as homogenous catalyst(s) from 2(1-cyclohexenyl)-cyclohexanone and RCONH₂ (US Patent 4006 236 (1977). The reaction time is longer, 10-24 hrs and the catalyst can not be reused and silica- alumina, chronia or magnesia catalyst(s) at comparatively high reaction temperature 180-425°C in vapour phase with low selectivity for a particular product. Our process using molecular sieve is eco-friendly, catalyst is reusable, separation is easy and selective.

### Objects of the invention

The main objective of the present invention is to provide process for the synthesis of octahydroacridine, octahydrophenanthridine and their derivatives, by using (modified or unmodified) zeolite or molecular sieve catalyst, which is an eco-friendly heterogeneous catalytic method.

Another objective of the invention is to improve yield and selectivity of the product.

### Summary of the invention

The present invention provides an improved process for the synthesis of annulated pyridines over a molecular sieve catalyst, the process comprising reacting a cyclic ketone having 5-8 carbons and an aliphatic aldehyde of the formula R¹CHO wherein R¹ is hydrogen or an alkyl group having 1 to 4 carbon atoms, with ammonia in an organic solvent in presence of a zeolite-type molecular sieve or heterogeneous catalyst, separating the catalyst to obtain the desired product.

In one embodiment of the invention, the cyclic ketone is cyclohexanone.

In another embodiment of the invention, the aliphatic aldehyde is selected from the group consisting of formaldehyde, acetaldehyde, propionaldehyde, and butyraldehyde.

In another embodiment of the invention, the molar ratio of cyclic ketone:aliphatic aldehyde: ammonia is in the range of 1:1:0.5 to 5.0.

In another embodiment of the invention, the reaction is carried out in liquid phase with a molar ratio of ammonia to cyclic ketone in the range of 0.5 to 5.0 and at a temperature in the range of 100°C to 250°C, for a period of 4-8hrs.

In another embodiment of the invention, the reaction is carried out in an autoclave.

In another embodiment of the invention, the organic solvent is selected from the group consisting of methanol, ethanol, isopropanol, acetonitrile and acetone, preferably methanol.

In another embodiment of the invention, the catalyst is selected from the group consisting of H-beta (Hβ), HY, HZSM-5 having a Si/Al ratio in the range of 40-300, H-mordenite, montmorillonite and SiO₂-Al₂O₃ zeolite and molecular sieve H-AIMCM-41.

In another embodiment of the invention, the ratio of Si to Al in the zeolite catalyst is in the range of 2.5-300.

In another embodiment of the invention, the catalyst is reusable.

In another embodiment of the invention, the annulated prydine obtained comprises 1,2,3,4,7,8,9,10-octahydrophenanthridine,1,2,3,4,5,6,7,8-octahydroacridine, 6-methyl octahydrophenanthridine, 6-ethyloctahydrophananthridine and 6-propyloctahydrophenanthridine.

### Detailed description of the invention

The present invention provides a process for the preparation of octahydroacridine of the formula number 1 to 9 below from cyclic ketones and aliphatic aldehyde with ammonia over molecular sieves. These annelated pyridines, like 9-amino-5,6,7,8-tetrahydroacridine (tacrine), are drug molecules or drug intermediates for various diseases like Alzheimer's disease, which is the most common cause of dementia in old people.

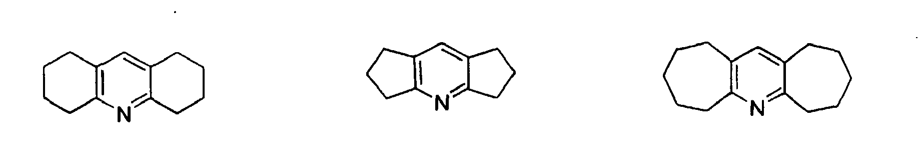

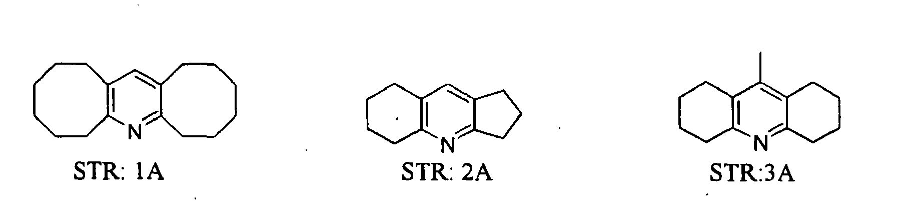

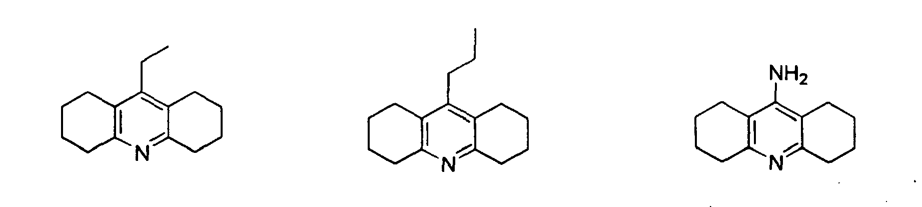

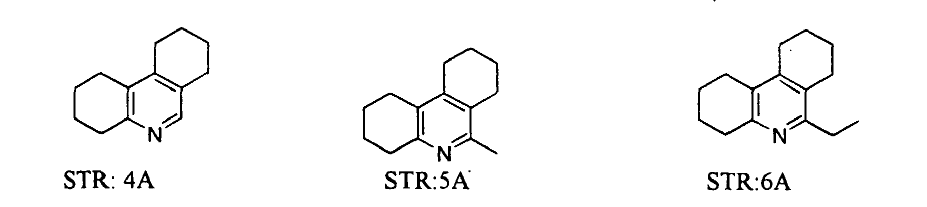

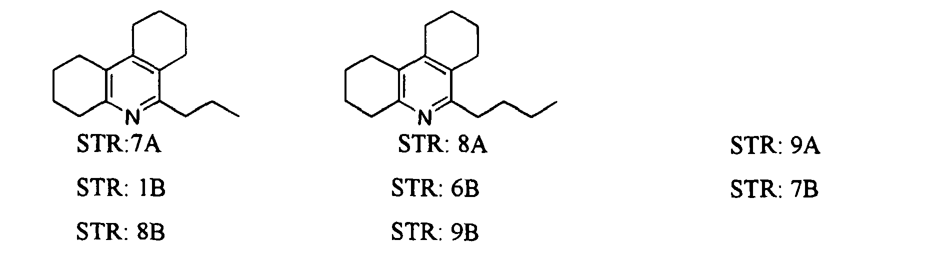

### Salient features of the process:

(a) The present invention provides a process that comprises of environmentally clean and economical technology and enables reusability of the catalyst;
(b) The process provides an eco-friendly method with high selectivity towards the product.
(c) The method provides a selective heterogeneous catalyst with longer life.
(d) This method provides a route, wherein the kind and composition of annulated pyridines can be varied by varying the starting materials.
(e) It also provides an efficient, economical method for synthesizing octahydroacridine and octahydrophenanthridine from cyclohexanone and formaldehyde with ammonia over various molecular sieve catalysts.

The present invention relates to a process for producing annulated pyridines by reacting a cyclic ketone and an aliphatic aldehyde with ammonia in liquid phase in the presence of a commercial or synthesized catalyst.

The aliphatic cyclic ketone used in the present invention includes cyclohexanone, cyclopentanone, cycloheptanone and cyclooctanone. The aliphatic aldehyde includes formaldehyde, acetaldehyde propionaldehyde, butyraldehyde and formamide. The combination of different cyclic ketones and aliphatic aldehydes as starting materials determines the main compounds of the annulated pyridine to be produced. Typical examples are given in the Table 1.

**Table 1:**

| Aldehyde | Ketone | Main products formed |
|---|---|---|
| Formaldehyde | Cyclohexanone | Octahydroacridine, Octahydrophenanthridine |
| Acetaldehyde | Cyclohexanone | 6-methyloctahydrophenanthridine |
| Propionaldehyde | Cyclohexanone | 6-ethyloctahydrophenanthridine |
| Butyraldehyde | Cyclohexanone | 6-propyloctahydrophenanthridine |
| Valaraldehyde | Cyclohexanone | 6-butyloctahydrophenanthridine |
| Formaldehyde | Cyclopentanone | Bis-bicyclopentylpyridine |
| Formaldehyde | Cycloheptanone | Bis-bicycloheptylpyridine |

The reaction of the present invention may be conducted in a batch-mode in an autoclave.

The reaction in batch-mode were carried out in Parr autoclave(600 mL) in the reaction temperature range of 100-250°C under constant stirring (30-60 revolutions per min). The typical molar ratio of cyclohexanone: formaldehyde:NH₃ was 1:1:3 and 222 mL methanol was used as solvent. The autoclave time was 6h.

A combination of cyclic ketone, aliphatic aldehyde and ammonia (or nitrogen source), for production of octahydroacridine and octahydrophenanthridine, was taken in the molar ratio of 1:1:0.5-5. The reaction can be effected without trouble or without much coking if the (liquid) starting materials contains water, methanol or the like solvent. Formaldehyde can be used in the form of formalin. The amount of coke deposited was less so higher yields of the products were obtained; in comparison with other commercial processes. The coke can be removed by heating the catalyst at 450°C to 550°C; for about 4-10 h.

The present invention is described below with reference to the following illustrative and non-limiting examples.

### EXAMPLE 1

The reaction of cyclohexanone, formaldehyde and ammonia was carried out in an autoclave (600ml) in presence of methanol as a solvent. Hβ (H-beta) was used as catalyst. The reaction was carried out in the temperature range of 100°Cto 215°C for 6h. The amount of catalyst was 2g. The molar ratio of cyclohexanone: formaldehyde: ammonia was 1:1:3. The liquid: catalyst ratio was 104.8 by weight. In a typical experiment the reaction (autoclavation) temperature was 150°C. The selectivities of octahydrophenanthridine were 50.2, 60.5, 44.8 and 32.2 percent at 53.0, 83.0, 97.7 and 98.7 percent conversions of cyclohexanone at 100°(4), 150°(14), 170°(20) and 215°C(49 atm) reaction temperature respectively. The selectivities of octahydroacridine were 7.7, 21.7, 39.4 and 42.2 percent respectively. With the increase of the autoclavation temperature the autogeneous pressure increased. With increase of autoclavation temperature the conversion and selectivities for octahydroacridine increased while the selectivities for octahydrophenanthridine decreased. The other products were cyclohexamine and cyclohexanoneoxime. Under similar to the supercritical conditions and with the increase of reaction temperature, there is substantial increase of reactive collisions, miscibility is increased, the mass transfer and heat transfer effects are enhanced resulting into increased yield of preferably of octahydroacridine using active zeolite catalyst.

### EXAMPLE 2

The reaction was carried out as explained in Example 1, with HZSM-5 catalyst. The autoclavation temperature was 150°C. The selectivities of octahydrophenanthridine and octahydroacridine were 54.6 and 28.8 percent at 80.3 percent conversion of cyclohexanone, over HZSM-5 (SiO₂/Al₂O₃ = 40).

### EXAMPLE 3

Reaction was carried out as described in Example 1 with HY zeolite as a catalyst. The selectivities for octahydrophenanthridine and octahydroacridine were 62.3% and 31.2% at 57.2% conversion of cyclohexanone over HY zeolite. Autoclavation temperature was 150°C.

### EXAMPLE 4

The reaction was carried out as described in Example 1, with H-mordenite as a catalyst. The selectivities of octahydrophenanthridine and octahydroacridine were 70.8% 19.8% at 89.1% conversion of cyclohexanone over H-mordenite zeolite. The autoclavation temperature was 150°C.

### EXAMPLE 5

The reaction of cyclohexanone, formaldehyde and ammonia was carried out as described in Example 1, with HMCM-41 (SiO₂/Al₂O₃ =30) as a catalyst. The selectivities of octahydrophenanthridine and octahydroacridine were 64.6% and 24.1% at 91.6% conversion of cyclohexanone over HMCM-41 mesoporous molecular sieve catalyst. The autoclavation temperature was 150°C.

### EXAMPLE 6

The reaction of cyclohexanone, formaldehyde and ammonia was carried out as described in Example 1, with HZSM-5 (SiO₂/Al₂O₃ = 40-280) as a catalyst. The selectivities of octahydrophenanthridine were 40.4%, 48.7% and 54.6% at 71.3%, 81.0% and 80.3% conversions of cyclohexanone over HZSM-5 (SiO₂/Al₂O₃= 280) HZSM-5 (150) and HZSM-5 (40) zeolites respectively. The selectivities of octahydroacridine were 10.6%, 18.4% and 28.8% respectively. The autoclavation temperature was 150°C

### EXAMPLE 7

The reactions of cyclohexanone, formaldehyde and ammonia were carried out as described in example 1, with H-beta (1-4 gm) as a catalyst. The selectivities of octahydrophenanthridine were 38.4,60.6 and 73.6 percent at 75.6, 83.0 and 94.4 percent conversions of cyclohexanone over H-beta(1gm), H-beta(2gm) and H-beta(4gm) zeolites respectively. The selectivities of octahydroacridine were 12.6, 21.7 and 19.7 percent respectively.The autoclavation temperature was 150°C

### EXAMPLE 8

The reactions of cyclohexanone, formaldehyde and ammonia were carried out as described in example 1, with H-beta (2gm) as a catalyst. The effect of solvent has been studied. The amount of solvent was 222ml. The selectivities of octahydrophenanthridine were 60.6, 44.1, 37.7, 20.4 and 8.4 percent at 83.0, 84.4, 77.7, 76.4 and 83.6 percent conversions of cyclohexanone for methanol (p=14atm), ethanol (p=11atm), acetonitrile (p=10atm) and acetone (p=13atm) as a solvent respectively. The corresponding selectivities for octahydroacridine were 21.7, 38.3, 40.1, 12.9 and 2.2 percent respectively. The molar ratio of cyclohexanone:formaldehyde:ammonia was 1:1:3.

### EXAMPLE 9

The reaction of cyclohexanone, formaldehyde and ammonia was carried out as described in example 1 with H-beta (2gm) as a catalyst the reaction was carried out at 228°C autoclavation (reaction) temperature and pressure was 64 atm. The reaction conditions are similar to the supercritical conditions for 6 hr using methanol (220 ml) as a solvent. The selectivities for octahydrophenanthridine and octahydroacridine were 23.9 and 40.6 percent at 98.4 percent conversion of cyclohexanone.

### EXAMPLE 10

The reactions of cyclohexanone, aliphatic aldehyde and ammonia were carried out as described in example 1, with H-beta (2 gm) as a catalyst. The reaction was carried out at 150°C using methanol (220ml) as a solvent and the molar ratio of cyclohexanone: aliphatic aldehyde: ammonia was 1:1:3. The selectivities for 6-methyloctahydrophenanthridine, 6-ethyloctahydrophenanthridine, 6-propyloctahydrophananthridine and 6-butyloctahydrophenanthridine were 80.9, 45.4, 63.2 and 27.7 percent at 97.0, 90.0, 96.4 and 93.5 percent conversions of cyclohexanone respectively.

### EXAMPLE 11

The reactions of cyclohexanone, acetaldehyde and ammonia were carried out as described in example 1 using methanol (220ml) as a solvent; by varying the various zeolites as catalysts. The reactions were carried out at 150°C and the molar ratio of cyclohexanone: acetaldehyde: ammonia was 1:1:3. The selectivities for 6-methylphenanthridine were 80.9, 39.5, 54.3, 32.2, 56.9, 79.8, 47.1, 48.4 percent at 97.0, 84.9, 94.9, 87.3, 96.7, 95.8, 87.7 and 90.3 percent conversions of cyclohexanone for H-beta, HZSM-5 (SiO₂/Al₂O₃=280), HZSM-5(40), HY, H-mordenite, HMCM-41(SiO₂/Al₂O₃=30), Montmorillonite (K-10) and SiO₂-Al₂O₃ (amorphous) catalysts respectively.

### EXAMPLE 12

The reactions of cyclohexanone, propionaldehyde and ammonia were carried out as described in example 1, using methanol (220ml) as a solvent; by varying the particularly zeolites as catalysts. The reactions were carried out at 150°C and the molar ratio of cyclohexanone: propionaldehyde: ammonia was 1:1:3. The selectivities for 6-ethyloctahydrophenanthridine were 45.4, 41.8, 37.7, 35.3, 39.0, 43.4, 41.5, 51.3, 28.4 percent at 90.0, 77.4, 83.3, 75.5, 80.0, 90.7, 78.2, 92.8 and 89.1 percent conversions of cyclohexanone using H-beta, HZSM-5(SiO₂/Al₂O₃=300), HZSM-5(SiO₂/Al₂O₃=150), HZSM-5(SiO₂/Al₂O₃=40), HY, H-mordenite, HMCM-41(SiO₂/Al₂O₃=30), montmorillonite and SiO₂-Al₂O₃(amorphous) catalysts respectively.

### EXAMPLE 13

The reactions of cyclohexanone, butyraldehyde and ammonia were carried out as described in example 1, using methanol (220ml) as a solvent by varying particularly various zeolites (2gm) as catalysts. The reactions were carried out at 150°C and the molar ratio of cyclohexanone: butyraldehyde: ammonia was 1:1:3. The selectivities for 6-propylphenanthridine were 46.8, 47.0, 36.5, 63.2 and 42.1 percent at 95.8, 95.7, 91.1, 96.4 and 95.6 percent conversions of cyclohexanone using HZSM-5 (SiO₂/Al₂O₃=150), HZSM-5(40), HY, H-mordenite and NaY zeolite respectively. The main by-product was cyclohexaneoxime.

## Claims

1. A process for the synthesis of annulated pyridines, the process comprising reacting a cyclic ketone having 5-8 carbons and an aliphatic aldehyde of the formula R¹CHO wherein R¹ is hydrogen or an alkyl group having 1 to 4 carbon atoms, with ammonia in an organic solvent in presence of a zeolite-type molecular sieve catalyst, separating the catalyst to obtain the desired product.

2. A process as claimed in claim 1, wherein the cyclic ketone is selected from the group consisting of cyclohexanone, cyclopentanone, cycloheptanone and cyclooctanone.

3. A process as claimed in claim 1, wherein the aliphatic aldehyde is selected from the group consisting of formaldehyde, acetaldehyde, propionaldehyde, butyraldehyde and formamide.

4. A process as claimed in claim 1, wherein the cyclic ketone is cyclohexanone.

5. A process as claimed in claim 1, wherein the molar ratio of cyclic ketone: aliphatic aldehyde:ammonia is in the range of 1:1:0.5 to 5.0.

6. A process as claimed in claim 1, wherein the reaction is carried out in liquid phase with a molar ratio of ammonia to cyclic ketone in the range of 0.5 to 5.0 and at a temperature in the range of 100°C to 250°C, for a period of 4-8hrs.

7. A process as claimed in claim 1, wherein the reaction is carried out in an autoclave.

8. A process as claimed in claim 1, wherein the organic solvent is selected from the group consisting of methanol, ethanol, isopropanol, acetonitrile and acetone.

9. A process as claimed in claim 1, wherein the solvent is methanol.

10. A process as claimed in claim 1, wherein the catalyst is selected from the group consisting of H-beta (Hβ), HY, HZSM-5 having a Si/Al ratio in the range of 40-300, H-mordenite, montmorillonite and SiO₂-Al₂O₃ zeolite and molecular sieve H-AlMCM-41.

11. A process as claimed in claim 1, wherein the ratio of Si to Al in the zeolite catalyst is in the range of 2.5 -300.

12. A process as claimed in claim 1, wherein the catalyst is reusable.

13. A process as claimed in claim 1, wherein the annulated pyrridine obtained comprises 1,2,3,4,7,8,9,10-octahydrophenanthridine,1,2,3,4,5,6,7,8-octahydroacridine, 6-methyl octahydrophenanthridine, 6-ethyloctahydrophenanthridine and 6-propyloctahydrophenanthridine.

## Patentansprüche

1. Verfahren zur Synthese von anellierten Pyridinen, wobei das Verfahren umfasst die Reaktion eines zyklischen Ketons mit 5-8 Kohlenstoffatomen und eines aliphatischen Aldehyds der Formel R¹CHO, wobei R¹ Wasserstoff oder eine Alkylgruppe mit 1 bis 4 Kohlenstoffatomen ist, mit Ammoniak in einem organischem Lösungsmittel in der Gegenwart eines Zeolith-Typ-Molekularsieb-Katalysators, Abtrennen des Katalysators, um das gewünschte Produkt zu erhalten.

2. Verfahren gemäß Anspruch 1, wobei das zyklische Keton ausgewählt wird aus der Gruppe bestehend aus Cyclohexanon, Cyclopentanon, Cycloheptanon und Cyclooctanon.

3. Verfahren gemäß Anspruch 1, wobei der aliphatische Aldehyd ausgewählt wird aus der Gruppe bestehend aus Formaldehyd, Acetaldehyd, Propionaldehyd, Butyraldehyd und Formamid.

4. Verfahren gemäß Anspruch 1, wobei das zyklische Keton Cyclohexanon ist.

5. Verfahren gemäß Anspruch 1, wobei das molare Verhältnis von zyklischem Keton:aliphatischem Aldehyd:Ammoniak im Bereich von 1:1:0,5 bis 5,0 liegt.

6. Verfahren gemäß Anspruch 1, wobei die Reaktion in flüssiger Phase mit einem molarem Verhältnis von Ammoniak zu zyklischem Keton im Bereich von 0,5 bis 5,0 und bei einer Temperatur im Bereich von 100°C bis 250°C für einen Zeitraum von 4-8 Stunden durchgeführt wird.

7. Verfahren gemäß Anspruch 1, wobei die Reaktion in einem Autoklaven durchgeführt wird.

8. Verfahren gemäß Anspruch 1, wobei das organische Lösungsmittel ausgewählt wird aus der Gruppe bestehend aus Methanol, Ethanol, Isopropanol, Acetonitril und Aceton.

9. Verfahren gemäß Anspruch 1, wobei das Lösungsmittel Methanol ist.

10. Verfahren gemäß Anspruch 1, wobei der Katalysator ausgewählt wird aus der Gruppe bestehend aus H-beta (Hβ), HY, HZSM-5, das ein Si/Al Verhältnis im Bereich von 40-300 aufweist, H-Mordenit, Montmorillonit und SiO₂-Al₂O₃ Zeolith und Molekularsieb H-AlMCM-41.

11. Verfahren gemäß Anspruch 1, wobei das Verhältnis von Si zu Al in dem ZeolithKatalysator im Bereich von 2,5-300 ist.

12. Verfahren gemäß Anspruch 1, wobei der Katalysator wieder verwendbar ist.

13. Verfahren gemäß Anspruch 1, wobei das resultierende anellierte Pyridin 1,2,3,4,7,8,9,10-Octahydrophenanthridin, 1,2,3,4,5,6,7,8-Octahydroacridin, 6-Methyloctahydrophenanthridin, 6-Ethyloctahydrophenanthridin und 6-Propyloctahydrophenanthridin umfasst.

## Revendications

1. Procédé de synthèse de pyridines annelées, le procédé comprenant la mise à réagir d'une cétone cyclique ayant 5 à 8 carbones et d'un aldéhyde aliphatique de formule R¹CHO dans laquelle R¹ est l'hydrogène ou un groupe alkyle ayant 1 à 4 atomes de carbones, avec de l'ammoniac dans un solvant organique en présence d'un catalyseur à tamis moléculaire de type zéolite, la séparation du catalyseur pour obtenir le produit souhaité.

2. Procédé selon la revendication 1, dans lequel la cétone cyclique est sélectionnée dans le groupe constitué par le cyclohexanone, le cyclopentanone, le cycloheptanone et le cyclooctanone.

3. Procédé selon la revendication 1, dans lequel l'aldéhyde aliphatique est sélectionné dans le groupe constitué par le formaldéhyde, l'acétaldéhyde, le propionaldéhyde, le butyraldéhyde et le formamide.

4. Procédé selon la revendication 1, dans lequel la cétone cyclique est le cyclohexanone.

5. Procédé selon la revendication 1, dans lequel le rapport molaire cétone cyclique:aldéhyde aliphatique:ammoniac est dans la plage de 1:1:0,5 à 5,0.

6. Procédé selon la revendication 1, dans lequel la réaction est réalisée en phase liquide avec un rapport molaire de l'ammoniac sur la cétone cyclique dans la plage de 0,5 à 5,0 et à une température dans la plage de 100°C à 250°C, pendant une période de 4 à 8 heures.

7. Procédé selon la revendication 1, dans lequel la réaction est réalisée dans un autoclave.

8. Procédé selon la revendication 1, dans lequel le solvant organique est sélectionné dans le groupe constitué par le méthanol, l'éthanol, l'isopropanol, l'acétonitrile et l'acétone.

9. Procédé selon la revendication 1, dans lequel le solvant est le méthanol.

10. Procédé selon la revendication 1, dans lequel le catalyseur est sélectionné dans le groupe constitué par H-bêta (Hβ), HY, HZSM-5 ayant un rapport Si/Al dans la plage de 40 à 300, la H-mordénite, la montmorillonite et la zéolite SiO₂-Al₂O₃ et le tamis moléculaire H-AlMCM-41.

11. Procédé selon la revendication 1, dans lequel le rapport de Si sur Al dans le catalyseur zéolite est dans la plage de 2,5 à 300.

12. Procédé selon la revendication 1, dans lequel le catalyseur est réutilisable.

13. Procédé selon la revendication 1, dans lequel la pyridine annelée obtenue comprend la 1,2,3,4,7,8,9,10-octahydrophénanthridine, la 1,2,3,4,5,6,7,8-octahydroacridine, la 6-méthyloctahydrophénanthridine, la 6-éthyloctahydrophénanthridine et la 6-propyloctahydrophénanthridine.
